# EUROPÄISCHE PATENTSCHRIFT

(11) **EP 1 107 941 B1**
(45) Veröffentlichungstag und Bekanntmachung des Hinweises auf die Patenterteilung: **17.07.2002**
(21) Anmeldenummer: 99942875.8
(22) Anmeldetag: 17.08.1999
(51) Int. Cl.: C07C 209/48, C07C 253/30

(54) **VERBESSERTES VERFAHREN ZUR GLEICHZEITIGEN HERSTELLUNG VON 6-AMINOCAPRONITRIL UND HEXAMETHYLENDIAMIN**
IMPROVED METHOD FOR SIMULTANEOUS PREPARATION OF 6-AMINOCAPRONITRILE AND HEXAMETHYLENE DIAMINE
PROCEDE AMELIORE DE PREPARATION SIMULTANEE DE 6-AMINOCAPRONITRILE ET DE DIAMINE D'HEXAMETHYLENE

(30) Priorität: 28.08.1998 DE 19839338
(43) Veröffentlichungstag der Anmeldung: 20.06.2001
(73) Patentinhaber: BASF AKTIENGESELLSCHAFT, 67056 Ludwigshafen (DE)
(72) Erfinder: LUYKEN, Hermann, D-67069 Ludwigshafen (DE); OHLBACH, Frank, D-69221 Dossenheim (DE); ANSMANN, Andreas, D-69168 Wiesloch (DE); BASSLER, Peter, D-68519 Viernheim (DE); FISCHER, Rolf, D-69121 Heidelberg (DE); MELDER, Johann-Peter, D-67459 Böhl-Iggelheim (DE); MERGER, Martin, D-67227 Frankenthal (DE); REHFINGER, Alwin, D-67112 Mutterstadt (DE); VOIT, Guido, D-67251 Freinsheim (DE); ACHHAMMER, Günther, D-68309 Mannheim (DE)
(86) Internationale Anmeldenummer: EP9906011
(87) Internationale Veröffentlichungsnummer: WO0012459

(56) Entgegenhaltungen:
- DE-A- 19 548 289
- DE-A- 19 636 765
- DE-A- 19 636 766
- DE-A- 19 704 612
- DE-A- 19 704 614
- DE-C- 19 614 154

## Beschreibung

Die vorliegende Erfindung betrifft ein Verfahren zur gleichzeitigen Herstellung von 6-Aminocapronitril und Hexamethylendiamin, ausgehend von Adipodinitril, umfassend die Schritte
a) Hydrierung von Adipodinitril in Gegenwart eines Katalysators, der als katalytisch aktive Komponente ein Element der achten Nebengruppe enthält, unter Erhalt einer Mischung enthaltend 6-Aminocapronitril, Hexamethylendiamin, Adipodinitril und Hochsieder,
b) Destillative Abtrennung von Hexamethylendiamin aus der Mischung enthaltend 6-Aminocapronitril, Hexamethylendiamin, Adipodinitril und Hochsieder, und entweder
c1) Destillative Abtrennung von 6-Aminocapronitril, und anschließend
d1) Destillative Abtrennung von Adipodinitril oder
c2) Gleichzeitige destillative Abtrennung von 6-Aminocapronitril und Adipodinitril in getrennte Fraktionen,
dadurch gekennzeichnet, daß in den Schritten dl) oder c2) die Sumpftemperaturen unterhalb von 185°C liegen.

Es ist bekannt, Adipodinitril (ADN) in Gegenwart von Elementen der achten Nebengruppe, insbesondere in Gegenwart von überwiegend Eisen, Cobalt, Nickel, Ruthenium oder Rhodium enthaltenden Katalysatoren, Lösungsmitteln wie z. B. Ammoniak, Aminen oder Alkoholen, und gegebenenfalls Zusätzen wie z. B. anorganischen Basen zu Gemischen aus 6-Aminocapronitril, Hexamethylendiamin und nicht umgesetztem Adipodinitril zu hydrieren. Dabei werden in der Flüssigphase homogen gelöste Katalysatoren und als Festbett oder in Suspension eingesetzte Festbettkatalysatoren verwendet.

Eisenkatalysatoren, die im allgemeinen als Festbettkatalysatoren bei hohem Druck in der Flüssigphase eingesetzt werden, sind z. B. in DE 4.235.466, WO 96/20.166, WO 96/20.043 und DE 19.636.767 beschrieben. Co-Katalysatoren sind z. B. aus DE 954.416, WO 96/20.166 und DE 19.636.768 bekannt. Nickelkatalysatoren werden nach DE 848.654 z. B. als Trägerkatalysatoren (Nickel auf Al₂O₃), vor allem aber z. B. nach US 2.762.835, WO 96/18.603 und WO 97/10.052 in Form von undotiertem oder dotiertem Raney-Nickel eingesetzt. Ruthenium-Festbettkatalysatoren sind aus US 3.322.815, homogen gelöste Rutheniumkatalysatoren aus WO 96/23.802 und WO 96/23.804 bekannt. Rhodiumkatalysatoren, wie z. B. Rhodium auf Magnesiumoxid, sind z. B. in US 4.601.859 genannt.

Die partielle Hydrierung von Adipodinitril zu Gemischen aus 6-Aminocapronitril, Hexamethylendiamin und nicht umgesetztem Adipodinitril wird durchgeführt, um 6-Aminocapronitril und Hexamethylendiamin in einem gewünschten, durch geeignete Wahl der Reaktionsbedingungen einstellbaren Verhältnis zu gewinnen. 6-Aminocapronitril läßt sich, z. B. nach US 5.646.277, in der Flüssigphase in Gegenwart von oxidischen Katalysatoren zu Caprolactam cyclisieren. Caprolactam stellt das Vorprodukt für Nylon 6, Hexamethylendiamin eines der beiden Vorprodukte für die Herstellung von Nylon 6.6 dar.

Aus der DE-A 19.548.289 ist ein Verfahren zur gleichzeitigen Herstellung von 6-Aminocapronitril und Hexamethylendiamin durch Hydrierung von Adipodinitril in Gegenwart eines Katalysators bei Teilumsatz, die Abtrennung von Hexamethylendiamin und 6-Aminocapronitril aus der Mischung und Umsetzung von 6-Aminocapronitril zu Caprolactam sowie Rückführung eines im wesentlichen aus Adipodinitril bestehenden Teils in das Verfahren bekannt.

Nachteilig bei diesen Verfahren ist, daß das bei der Aufarbeitung des Reaktionsaustrags zurückgewonnene Adipodinitril unerwünschte Nebenprodukte enthält, insbesondere Amine wie z. B. 1-Amino-2-cyanocyclopenten (ACCPE) und Bishexamethylentriamin (BHMTA), die zu Ausbeuteverlusten an den Wertprodukten führen können.

Die Nebenprodukte lassen sich nach den beschriebenen Verfahren destillativ von Adipodinitril infolge der Bildung von Azeotropen oder Quasi-Azeotropen nicht abtrennen. Dies führt insbesondere bei einer Rückführung des Adipodinitrils zu einer Aufpegelung im Gesamtverfahren.

Bei dieser Rückführung kann sich aus ACCPE in dem Hydrierschritt das Folgeprodukt 2-Aminomethylcyclopentylamin (AMCPA) bilden, welches das Wertprodukt Hexamethylendiamin verunreinigt. Aus der US-A 3.696.153 ist bekannt, daß sich AMCPA nur sehr schwer von Hexamethylendiamin abtrennen läßt.

Aus DE 19.636.766 ist bekannt, dem rückzuführenden Adipodinitril 0,01 bis 10 Gew.-% einer Säure, bezogen auf Adipodinitril oder einen sauren Ionentauscher zuzusetzen, das Adipodinitril von diesem Gemisch abzutrennen und es in den Hydrierreaktor zurückzuführen. Hierbei werden stickstoffhaltige basische Nebenprodukte durch den Zusatz der Säuren neutralisiert. Nachteilig an dieser Methode ist die Bildung von Salzen, die aus dem Verfahren ausgeschleust und entsorgt werden müssen. Hierzu ist ein zusätzlicher Verfahrensschritt notwendig.

Aufgabe der vorliegende Erfindung war daher, ein Verfahren zur Verfügung zu stellen, das die Abtrennung von Adipodinitril aus einer durch partielle Hydrierung von Adipodinitril gewonnenen Mischung enthaltend Adipodinitril, Hexamethylendiamin, 6-Amincapronitril und Komponenten, die einen Siedepunkt über dem von Adipodinitril aufweisen ("Hochsieder"), auf technisch einfache und wirtschaftliche Weise unter Vermeidung der genannten Nachteile sowie die Rückgewinnung von möglichst reinem, insbesondere wenig ACCPE enthaltendem, Adipodinitril ermöglicht.

Diese Aufgabe wurde gelöst durch das eingangs definierte Verfahren.

Das in dem erfindungsgemäßen Verfahren eingesetzte Adipodinitril kann im allgemeinen nach den an sich bekannten Verfahren, vorzugsweise durch Umsetzung von Butadien mit Blausäure in Gegenwart von Katalysatoren, insbesondere Nickel-(0)-Komplexverbindungen und phosphorhaltigen Cokatalysatoren, über Pentennitril als Zwischenstufe, hergestellt werden.

In einer bevorzugten Ausführungsform sollte der Gehalt an 1-Amino-2-cyano-cyclopenten, bezogen auf Adipodinitril, in dem in Schritt a) eingesetzten Adipodinitril unter 5000 Gew.-ppm, vorteilhaft zwischen 10 und 5000 Gew.-ppm, bevorzugt zwischen 10 und 3000 Gew.-ppm, besonders bevorzugt zwischen 10 und 1500 Gew.-ppm, insbesondere zwischen 10 und 100 Gew.-ppm liegen.

Hierdurch kann die Ausbeute an 6-Aminocapronitril und Hexamethylendiamin gesteigert und die Reinigung von Hexamethylendiamin erleichtert werden.

Die partielle Hydrierung von Adipodinitril kann man nach einem der bekannten Verfahren durchführen, beispielsweise nach einem der zuvor genannten Verfahren, beschrieben in US 4.601.859.1, US 2.762.835, US 2.208.598, DE-A 848.654, DE-A 95.44.161, WO 96/18.603, WO 97/10.052, DE-A 42.35.466 oder WO 92/21.650, indem man im allgemeinen die Hydrierung in Gegenwart von einem Element der achten Nebengruppe oder deren Gemische, wie Nickel-, Cobalt-, Eisen-, Ruthenium- oder Rhodium-haltigen Katalysatoren durchführt. Dabei können die Katalysatoren als homogen gelöste Katalysatoren oder suspendierte oder fest angeordnete Trägeroder Vollkatalysatoren verwendet werden. Als Katalysatorträger kommen beispielsweise Aluminiumoxid, Siliciumdioxid, Titandioxid, Magnesiumoxid, Aktivkohlen und Spinelle in Frage. Als Vollkatalysatoren kommen beispielsweise Raney-Nickel und Raney-Cobalt in Betracht, die noch durch weitere Elemente dotiert sein können.

Üblicherweise wählt man die Katalysatorbelastung im Bereich von 0,05 bis 10 kg, vorzugsweise von 0,1 bis 5 kg Adipodinitril/l Kat. x h.

Die Hydrierung nimmt man in der Regel bei Temperaturen im Bereich von 20 bis 220 °C, vorzugsweise von 50 bis 150 °C, und bei Wasserstoff-Partialdrücken von 0,1 bis 40 MPa, vorzugsweise von 0,5 bis 30 MPa vor.

Bevorzugt führt man die Hydrierung in Gegenwart eines Lösungsmittels wie Ammoniak, Aminen oder Alkoholen, insbesondere Ammoniak, durch. Die Ammoniak-Menge wählt man im allgemeinen im Bereich von 0,1 bis 10 kg, vorzugsweise 0,5 bis 3 kg Ammoniak/kg Adipodinitril.

Das Molverhältnis von 6-Aminocapronitril zu Hexamethylendiamin und damit das Molverhältnis von Caprolactam zu Hexamethylendiamin kann durch den jeweils gewählten Adipodinitril-Umsatz gesteuert werden. Bevorzugt arbeitet man bei Adipodinitril-Umsätzen im Bereich von 10 bis 90 %, bevorzugt von 30 bis 80 %, um hohe 6-Aminocapronitril-Selektivitäten zu erhalten.

In der Regel liegt die Summe aus 6-Aminocapronitril und Hexamethylendiamin je nach Katalysator und Reaktionsbedingungen bei 95 bis 99 %, wobei als mengenmäßig bedeutendstes Nebenprodukt Hexamethylenimin auftritt.

Als Katalysatoren setzt man bevorzugt Nickel-, Ruthenium-, Rhodium-, Eisen- und Cobalt-haltige Verbindungen ein, bevorzugt solche vom Raney-Typ, insbesondere Raney-Nickel und Raney-Cobalt. Man kann die Katalysatoren auch als Trägerkatalysatoren einsetzen, wobei als Träger beispielsweise Aluminiumoxid, Siliciumdioxid, Zinkoxid, Aktivkohle oder Titandioxid dienen können (S. Appl. Het. Cat., 1987, S. 106 bis 122; Catalysis, Vol. 4 (1981) S. 1 bis 30). Besonders bevorzugt ist Raney-Nickel.

Die Nickel-, Ruthenium-, Rhodium-, Eisen- und Cobalt-Katalysatoren können vorteilhaft mit Metallen der Gruppe VIB (Cr, Mo, W) und VIII (Fe, Ru, Os, Co (nur im Falle von Nickel), Rh, Ir, Pd, Pt) des Periodensystems modifiziert sein. Nach bisherigen Beobachtungen, beispielsweise gemäß DE-A 22.60.978; Bull. Soc. Chem. 13 (1946) S 208), führt der Einsatz von insbesondere modifizierten Raney-Nickel-Katalysatoren, beispielsweise mit Chrom und/oder Eisen modifiziert, zu höheren 6-Aminocapronitril-Selektivitäten.

Die Menge an Katalysator wählt man im allgemeinen so, daß die Cobalt-, Ruthenium-, Rhodium-, Eisen- oder Nickel-Menge im Bereich von 1 bis 50 Gew.-%, bevorzugt von 5 bis 20 Gew.-%, bezogen auf die eingesetzte Menge an Dinitril, beträgt.

Die Katalysatoren können als Festbettkatalysatoren in Sumpf- oder Rieselfahrweise oder als Suspensionskatalysatoren eingesetzt werden.

In einer weiteren bevorzugten Ausführungsform hydriert man Adipodinitril partiell zu 6-Aminocapronitril bei erhöhter Temperatur und erhöhtem Druck in Gegenwart eines Lösungsmittels und eines Katalysators, indem man einen Katalysator verwendet, der
i) eine Verbindung auf der Basis eines Metalles, ausgewählt aus der Gruppe, bestehend aus Nickel, Cobalt, Eisen, Ruthenium und Rhodium,
ii) von 0,01 bis 25 Gew.-%, vorzugsweise von 0,1 bis 5 Gew.-% bezogen auf a), eines Promotors auf der-Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
iii) von 0 bis 5 Gew.-%, vorzugsweise von 0,1 bis 3 Gew.-%, bezogen auf i), einer Verbindung auf der Basis eines Alkalimetalles oder eines Erdalkalimetalles, enthält,
wobei vorzugsweise, wenn als Komponente i) eine Verbindung auf der Basis von nur Ruthenium oder Rhodium oder Ruthenium und Rhodium oder Nickel und Rhodium gewählt wird, der Promotor ii) gewünschtenfalls entfallen und weiterhin vorzugsweise die Komponente i) nicht auf der Basis von Eisen besteht, wenn die Komponente ii) Aluminium ist.

Bevorzugte Katalysatoren sind solche, in denen die Komponente i) mindestens eine Verbindung auf der Basis eines Metalles, ausgewählt aus der Gruppe aus Nickel, Cobalt und Eisen, in einer Menge im Bereich von 10 bis 95 Gew.-% sowie Ruthenium und/oder Rhodium in einer Menge im Bereich von 0,1 bis 5 Gew.-%, jeweils bezogen auf die Summe der Komponenten i) bis iii), enthält, die Komponente ii) mindestens einen Promotor auf der Basis eines Metalles, ausgewählt aus der Gruppe bestehend aus Silber, Kupfer, Mangan, Rhenium, Blei und Phosphor, in einer Menge im Bereich von 0,1 bis 5 Gew.-%, bezogen auf i), enthält, und die Komponente iii) mindestens eine Verbindung auf der Basis der Alkalimetalle und Erdalkalimetalle, ausgewählt aus der Gruppe bestehend aus Lithium, Natrium, Kalium, Cäsium, Magnesium und Calcium, in einer Menge im Bereich von 0,1 bis 5 Gew.-% enthält.

Besonders bevorzugte Katalysatoren sind solche, die
i) eine Verbindung auf der Basis von Eisen wie Eisenoxid enthalten und
ii) von 0 bis 5 Gew.-% bezogen auf i) eines Promotors auf der Basis eines Elementes oder 2, 3, 4, 5 oder 6 Elementen ausgewählt aus der Gruppe bestehend aus Aluminium, Silizium, Zirkonium, Vanadium, Mangan und Titan sowie
iii) von 0 bis 5 Gew.-%, vorzugsweise 0,1 bis 3 Gew.-%, insbesondere 0,1 bis 0,5 Gew.-% bezogen auf i) einer Verbindung auf der Basis eines Alkali- oder Erdalkalimetalles, vorzugsweise ausgewählt aus der Gruppe bestehend aus Lithium, Natrium, Kalium, Rubidium, Cäsium, Magnesium und Calcium.

Bei den bevorzugt einsetzbaren Katalysatoren kann es sich um Voll- oder Trägerkatalysatoren handeln. Als Trägermaterialien kommen beispielsweise poröse Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung erfolgt in der Regel derart, daß man Vorläufer der Komponenten a) zusammen mit Vorläufern der Promotoren, Komponenten ii), und gewünschtenfalls mit Vorläufern der Komponenten iii) in Gegenwart oder Abwesenheit von Trägermaterialien (je nachdem, welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten i), ii) und gewünschtenfalls iii) tränkt, wobei man die einzelnen Komponenten gleichzeitig oder nacheinander zugeben kann, oder indem man die Komponenten i), ii) und gewünschtenfalls iii) auf den Träger nach an sich bekannten Methoden aufsprüht.

Als Vorläufer der Komponenten i) kommen in der Regel gut wasserlösliche Salze der zuvor genannten Metalle wie Nitrate, Chloride, Acetate, Formiate und Sulfate in Betracht, vorzugsweise Nitrate.

Als Vorläufer der Komponente ii) kommen in der Regel gut wasserlösliche Salze oder Komplexsalze der zuvor genannten Metalle wie Nitrate, Chloride, Acetate, Formiate und Sulfate sowie insbesondere Hexachloroplatinat in Betracht, vorzugsweise Nitrate und Hexachloroplatinat.

Als Vorläufer der Komponenten iii) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150°C, vorzugsweise von 80 bis 120°C vor. Das Calcinieren nimmt man üblicherweise bei Temperaturen im Bereich von 150 bis 500°C, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 80 bis 250°C, vorzugsweise von 80 bis 180 °C bei Katalysatoren auf der Basis von Ruthenium oder Rhodium als Komponenten i), oder im Bereich von 200 bis 500°C, vorzugsweise von 250 bis 400°C bei Katalysatoren auf der Basis eines der Metalle ausgewählt aus der Gruppe aus Nickel, Cobalt und Eisen als Komponente i) 2 bis 24 Stunden einer Wasserstoff-Atmosphäre oder einer Gasmischung, enthaltend Wasserstoff und ein Inertgas wie Stickstoff, aussetzt. Die Katalysatorbelastung beträgt hierbei bevorzugt 200 l/l Katalysator.

Vorteilhaft führt man die Aktivierung des Katalysators direkt im Synthese-Reaktor durch, da hierdurch üblicherweise ein ansonsten erforderlicher Zwischenschritt, nämlich die Passivierung der Oberfläche bei üblicherweise Temperaturen im Bereich von 20 bis 80°C, vorzugsweise von 25 bis 35°C, mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500°C, vorzugsweise von 200 bis 350°C in einer wasserstoffhaltigen Atmosphäre vor.

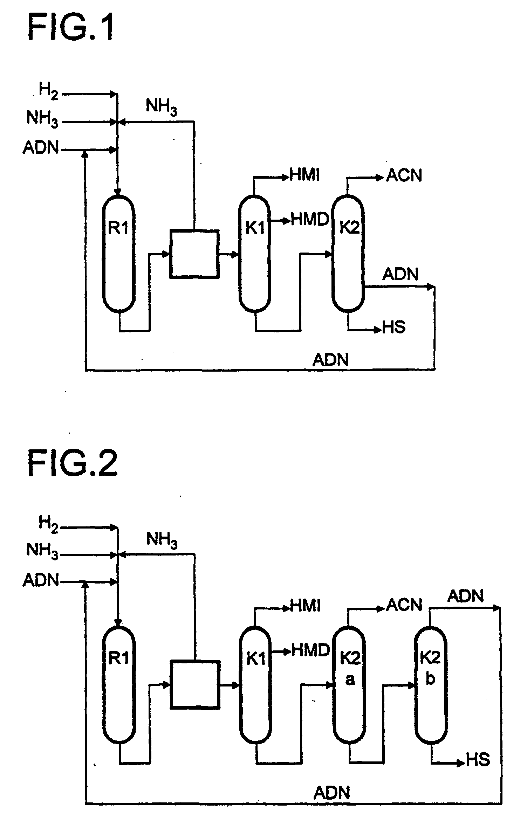

Die Katalysatoren können in einem Reaktor R1 als Festbettkatalystoren in Sumpf- oder Rieselfahrweise oder als Suspensionskatalysatoren eingesetzt werden (siehe Abbildung 1).

Führt man die Umsetzung in einer Suspension durch, wählt man üblicherweise Temperaturen im Bereich von 40 bis 150°C, vorzugsweise von 50 bis 100°C, besonders vorzugsweise von 60 bis 90°C; den Druck wählt man im allgemeinen im Bereich von 2 bis 30 MPa, vorzugsweise von 3 bis 30 MPa, besonders bevorzugt von 4 bis 9 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise im Bereich von 50 bis 275 Minuten, vorzugsweise von 70 bis 200 Minuten.

Bei der Suspensionsfahrweise kann man vorteilhaft insbesondere flüssige Verdünnungsmittel zusetzen, vorteilhaft primäre, sekundäre oder tertiäre Amine, wie Monoamine, Diamine und Triamine mit 1 bis 6 C-Atomen, beispielsweise Trimethylamin, Triethylamin, Tripropylamin und Tributylamin, oder Alkohole, insbesondere Methanol und Ethanol, bevorzugt Ammoniak, oder deren Gemische ein. Zweckmäßig wählt man eine Adipodinitril-Konzentration im Bereich von 10 bis 90 Gew.-%, vorzugsweise von 30 bis 80 Gew.-%, besonders bevorzugt von 40 bis 70 Gew.-%, bezogen auf die Summe von Adipodinitril und Verdünnungsmittel.

Die Menge an Katalysator wählt man im allgemeinen so, daß die Katalysatormenge im Bereich von 1 bis 50 Gew.-%, bevorzugt von 5 bis 20 Gew.-% bezogen auf die eingesetzte Menge an Adipodinitril, beträgt.

Man kann die partielle Hydrierung auch diskontinuierlich oder kontinuierlich an einem Festbettkatalysator in Riesel- oder Sumpffahrweise durchführen, wobei man üblicherweise eine Temperatur im Bereich von 20 bis 150°C, vorzugsweise von 30 bis 90°C, und einem Druck in der Regel im Bereich von 2 bis 40 MPa, vorzugsweise von 3 bis 30 MPa, wählt.

Vorteilhaft kann man insbesondere flüssige Verdünnungsmittel zusetzen, vorteilhaft primäre, sekundäre oder tertiäre Amine, wie Monoamine, Diamine und Triamine mit 1 bis 6 C-Atomen, beispielsweise Trimethylamin, Triethylamin, Tripropylamin und Tributylamin, oder Alkohole, insbesondere Methanol und Ethanol, bevorzugt Ammoniak, oder deren Gemische ein.

In einer bevorzugten Ausführungsform wählt man einen Gehalt an Ammoniak im Bereich von 1 bis 10 g, bevorzugt von 2 bis 6 g pro g Adipodinitril. Bevorzugt wählt man dabei eine Katalysatorbelastung im Bereich von 0,1 bis 2,0 kg, vorzugsweise von 0,3 bis 1,0 kg Adipodinitril/1 x h. Auch hier kann man durch Veränderung der Verweilzeit den Umsatz und damit die Selektivität gezielt einstellen.

Als vorteilhaft hat sich die Zugabe basischer Zusätze, insbesondere von Hydroxiden, Carbonaten oder Alkoholaten der Alkaili-oder Erdalkalimetalle oder Gemische solcher Verbindungen, bei der Hydrierung gemäß Schritt a) erwiesen.

Wurde in Schritt a) ein Verdünnungsmittel zugesetzt, so kann dieses vorteilhaft zwischen Schritt a) und Schritt b) in an sich bekannter Weise, vorzugsweise destillativ, abgetrennt und beispielsweise in Schritt a) erneut eingesetzt werden.

Der Hydrieraustrag enthält vor Schritt b) neben 6-Aminocapronitril, Hexamethylendiamin und Adipodinitril üblicherweise unter anderem Hexamethylenimin, Bis-hexamethylen-triamin und als Hochsieder 2-(5-Cyano-pentylamino)-tetrahydroazepin und 2-(6-Aminohexylamino)-tetrahydroazepin, also Stickstoffbasen.

Der Hydrieraustrag kann erfindungsgemäß in zwei Schrittfolgen destillativ aufgearbeitet werden (siehe Abbildungen 1 und 2).

Aus dem Reaktionsaustrag wird zunächst Hexamethylendiamin zusammen mit dem Nebenprodukt Hexamethylenimin abgetrennt (Schritt b)). Dies kann in zwei oder mehr Kolonnen, vorzugsweise einer Kolonne (K 1) erfolgen.

Aus Adipodinitril können in Gegenwart der in den Sumpfprodukten enthaltenen Stickstoffbasen erheblich größere Mengen an ACCPE entstehen als in Abwesenheit solcher Stickstoffbasen.

In einer bevorzugten Ausführungsform sollte die Sumpftemperatur in Schritt b) unter 185°C, vorzugsweise unter 180°C liegen, wobei sich wegen des geringen Dampfdrucks der zu trennenden Verbindungen eine Sumpftemperatur von mindestens 100°C, vorzugsweise mindestens 130°C empfiehlt. Die Drücke im Sumpf der Kolonne sollten vorteilhaft 0,1 bis 100, insbesondere 5 bis 40 mbar betragen. Vorzugsweise sollten die Verweilzeiten des Sumpfprodukte in der Destillation gemäß Schritt b) 1 bis 60, insbesondere 5 bis 15 Minuten betragen.

Das aus der Destillation gemäß Schritt b) erhaltene Sumpfprodukt kann auf zwei alternativen Wegen, Schrittfolge c1) und d1) oder Schritt c2) aufgearbeitet werden.

Gemäß Schritt c2 (Abbildung 1) wird das Sumpfprodukt einer Kolonne K 2 zugeführt, in der 6-Aminocapronitril über Kopf, Adipodinitril in einem Seitenabzug und Hochsieder über Sumpf abgetrennt werden.

Die Sumpftemperatur in Schritt c2) liegt erfindungsgemäß unter 185°C, vorzugsweise unter 180°C, wobei sich wegen des geringen Dampfdrucks der zu trennenden Verbindungen eine Sumpftemperatur von mindestens 100°C, vorzugsweise mindestens 130°C empfiehlt. Die Drücke im Sumpf der Kolonne sollten vorteilhaft 0,1 bis 100, insbesondere 5 bis 40 mbar betragen. Vorzugsweise sollten die Verweilzeiten des Sumpfprodukte in der Destillation gemäß Schritt c2) 1 bis 60, insbesondere 5 bis 15 Minuten betragen.

Gemäß Schrittfolge c1)/d1) (Abbildung 2) wird das Sumpfprodukt einer Kolonne K 2a zugeführt, in der man das 6-Aminocapronitril über Kopf destillativ abtrennt (Schritt c1)), das Sumpfprodukt einer Kolonne K 2b zugeführt und dort Adipodinitril über Kopf destillativ (Schritt d1)) und Hochsieder über Sumpf abtrennt.

In einer bevorzugten Ausführungsform sollte die Sumpftemperatur in Schritt c1) unter 185°C, vorzugsweise unter 180°C liegen, wobei sich wegen des geringen Dampfdrucks der zu trennenden Verbindungen eine Sumpftemperatur von mindestens 100°C, vorzugsweise mindestens 130°C empfiehlt. Die Drücke im Sumpf der Kolonne sollten vorteilhaft 0,1 bis 100, insbesondere 5 bis 40 mbar betragen. Vorzugsweise sollten die Verweilzeiten des Sumpfprodukte in der Destillation gemäß Schritt c1) 1 bis 60, insbesondere 5 bis 15 Minuten betragen.

Die Sumpftemperatur in Schritt dl) liegt vorteilhaft unter 185°C, vorzugsweise unter 180°C, wobei sich wegen des geringen Dampfdrucks der zu trennenden Verbindungen eine Sumpftemperatur von mindestens 100°C, vorzugsweise mindestens 130°C empfiehlt. Die Drücke im Sumpf der Kolonne sollten vorteilhaft 0,1 bis 100, insbesondere 5 bis 40 mbar betragen. Vorzugsweise sollten die Verweilzeiten des Sumpfprodukte in der Destillation gemäß Schritt d1) 1 bis 60, insbesondere 5 bis 15 Minuten betragen.

Zur weiteren Verringerung des Gehalts an Nebenprodukten, wie Stickstoffbasen, insbesondere Bis-hexamethylen-triamin und ACCPE, in dem zurückgewonnenen Adipodinitril kann in dem erfindungsgemäßen Verfahren vorteilhaft in den Sumpf der Kolonne K 2b diskontinuierlich oder vorzugsweise kontinuierlich eine organische oder anorganische Säure zugeführt werden oder das nach den Kolonnen K 2 oder K 2b erhaltene Adipodinitril diskontinuierlich oder vorzugsweise kontinuierlich mit einer organischen oder anorganischen Säure gereinigt werden.

Das nach beiden Alternativen erhaltene Adipodinitril kann vorteilhaft in der partiellen Hydrierung zu 6-Aminocapronitril und Hexamethylendiamin, beispielsweise durch Rückführung in Schritt a) des erfindungsgemäßen Verfahrens, oder in ein Verfahren zur vollständigen Hydrierung zu Hexamethylendiamin eingesetzt werden.

Überraschenderweise wurde bei der Rückführung des nach dem erfindungsgemäßen Verfahren gewonnenen Adipodinitrils in die partielle Hydrierung festgestellt, daß die erfindungsgemäße Absenkung der Menge an 1-Amino-2-cyanocyclopenten im rückgeführten Adipodinitril zu deutlichen Vorteilen bei der Hydrierung, der destillativen Reinigung von Hexamethylendiamin und der Standzeit des Hydrierkatalysators führt.

### Beispiel 1:

a) Herstellung eines Eisen-Hydrierkatalysators
   Für die partielle Hydrierung von Adipodinitril zu 6-Aminocapronitril und Hexamethylendiamin wurde ein Eisenkatalysator auf der Basis eines Magnetiterzes verwendet, der nach DE 19.636.767, Beispiel 2 a), hergestellt worden war. Verwendet wurde die Siebfraktion der Teilchengröße 3 bis 5 mm.
b) Partielle Hydrierung von Adipodinitril
   Ein Rohrreaktor (Länge 180 cm, d = 30 mm) wurde mit 720 ml (1630 g) der nach a) hergestellten Katalysatormasse befüllt und drucklos im Wasserstoffstrom (500 Nl/h) reduziert. Dabei wurde die Temperatur innerhalb von 24 Stunden von 30°C auf 340°C angehoben und anschließend 72 Stunden bei 340°C gehalten.
   Nach Absenken der Temperatur wurden dem Reaktor bei 250 bar und 90 °C Zulauftemperatur 330 g/h ADN (hergestellt aus Butadien und Blausäure in Gegenwart von Ni-(0)-Komplexen als Katalysator und Phosphorverbindungen Cokatalysatoren), 1200 g/h Ammoniak und 140 Nl/h Wasserstoff zugeführt.
   Die Hydrierung wurde 1500 Stunden lang unter den angegebenen Bedingungen betrieben. Dabei wurde über die gesamte Laufzeit bei einem ADN-Umsatz von 60 % eine konstante Gesamtselektivität (Summe der Selektivitäten von 6-Aminocapronitril und Hexamethylendiamin) von 99 % gefunden. Die 6-Aminocapronitril-Selektivität sank während der Laufzeit von 50 % auf 48,5 % ab.
c) Aufarbeitung des Hydrieraustrags
   Für die diskontinuierlich durchgeführte Aufarbeitung wurden Hydrierausträge während des Versuches gesammelt.
   Aus ihnen wurde zunächst der Ammoniak in einer Kolonne mit 20 theoretischen Böden über Kopf abdestilliert. Als Sumpfprodukt wurde ein Gemisch erhalten, das laut gaschromatographischer Analyse zu etwa 30 Mol-% aus 6-Aminocapronitril, zu 39 Mol-% aus Adipodinitril und zu 30 Mol-% aus Hexamethylendiamin bestand. Als mengenmäßig wichtigstes Nebenprodukt war Hexamethylenimin, weiterhin je 0,15 Mol-% 2-(5-Cyano-pentylamino)-tetrahydroazepin und 2-(6-Amino-hexylamino)-tetrahydroazepin enthalten.
   Aus 1000 g erhaltenem Sumpfprodukt wurden in der gleichen Kolonne über Kopf 296 g Hexamethylendiamin bei einer Sumpftemperatur von 180°C abgetrennt, das etwa 0,5 Gew.-% Hexamethylenimin enthielt.
   695 g des erhaltenen Sumpfprodukts wurden in einer kontinuierlich betriebenen Kolonne so destilliert, daß über Kopf rund 305 g 6-Aminocapronitril, über einen Seitenabzug 380 g Adipodinitril und über Sumpf 10 g Adipodinitril enthaltende Hochsieder abgezogen wurden. Destilliert wurde bei 20 bis 40 mbar Kopfdruck. Die Sumpftemperatur der Kolonne wurde durch Veränderung des Kopfdrucks variiert. Das Rücklaufverhältnis betrug 2 : 1. Tabelle 1 zeigt die Abhängigkeit der 1-Amino-2-cyano-cyclopenten-Menge im Adipodinitril des Seitenabzugs in Abhängigkeit von der Sumpftemperatur der Kolonne.

**Tabelle1**

| Sumpftemperatur (°C) | ACCPE¹⁾ (ppm) |
|---|---|
| 198,5 | 12300 |
| 184,9 | 11300 |
| 184,2 | 8900 |
| 182,2 | 3600 |
| 182,0 | 3200 |
| 180,9 | 2700 |
| 180,5 | 2700 |
| 180,0 | 2800 |
| 178,2 | 2800 |

| | |
|---|---|
| 1) ppm 1-Amino-2-cyanocyclopenten, bezogen auf im Seitenabzug erhaltenes Adipodinitril | |

### Vergleichsbeispiel

300 g ADN (hergestellt aus Butadien und Blausäure in Gegenwart von Ni-(0)-Komplexen als Katalysator und Phosphorverbindungen als Cokatalysatoren) mit einer Reinheit von 99,9 % und einem Gehalt an 1-Amino-2-cyano-penten von 21 Gew.-ppm, bezogen auf ADN, wurden bei einer Sumpftemperatur von 200-205°C, einem Druck von 70 mbar und einer Siedetemperatur von 200°C destilliert. Als Sumpfprodukt blieben nur Spuren an Hochsiedern zurück.

Das destillativ erhaltene ADN enthielt 138 Gew.-ppm 1-Amino-2-cyano-cyclopenten gemäß gaschromatographischer Analyse.

## Patentansprüche

1. Verfahren zur gleichzeitigen Herstellung von 6-Aminocapronitril und Hexamethylendiamin, ausgehend von Adipodinitril, umfassend die Schritte
a) Hydrierung von Adipodinitril in Gegenwart eines Katalysators, der als katalytisch aktive Komponente ein Element der achten Nebengruppe enthält, unter Erhalt einer Mischung enthaltend 6-Aminocapronitril, Hexamethylendiamin, Adipodinitril und Hochsieder,
b) Destillative Abtrennung von Hexamethylendiamin aus der Mischung enthaltend 6-Aminocapronitril, Hexamethylendiamin, Adipodinitril und Hochsieder, und entweder
c1) Destillative Abtrennung von 6-Aminocapronitril, und anschließend
d1) Destillative Abtrennung von Adipodinitril oder
c2) Gleichzeitige destillative Abtrennung von 6-Aminocapronitril und Adipodinitril in getrennte Fraktionen,
**dadurch gekennzeichnet, daß** in den Schritten d1) oder c2) die Sumpftemperaturen unterhalb von 185°C liegen.

2. Verfahren nach Anspruch 1, wobei in den Schritten d1) oder c2) die Sumpftemperaturen unterhalb von 180°C liegen.

3. Verfahren nach Anspruch 1 oder 2, wobei der Katalysator in Schritt a) als katalytisch aktives Element Eisen, Cobalt, Nickel, Ruthenium oder Rhodium oder deren Gemische enthält.

4. Verfahren nach den Ansprüchen 1 bis 3, wobei der Katalysator in Schritt a) als katalytisch aktives Element Eisen, Cobalt oder Nickel oder deren Gemische enthält.

5. Verfahren nach den Ansprüchen 1 bis 4, wobei der Katalysator in Schritt a) auf Raney-Nickel oder Raney-Cobalt oder deren Gemische basiert.

6. Verfahren nach den Ansprüchen 1 bis 4, wobei man in Schritt a) einen Katalysator verwendet, der
i) eine Verbindung auf der Basis eines Metalles, ausgewählt aus der Gruppe, bestehend aus Nickel, Cobalt, Eisen, Ruthenium und Rhodium,
ii) von 0,01 bis 25 Gew.-%, vorzugsweise von 0,1 bis 5 Gew.-%, bezogen auf a) eines Promotors auf der Basis eines Metalles, ausgewählt aus der Gruppe, bestehend aus Palladium, Platin, Iridium, Osmium, Kupfer, Silber, Gold, Chrom, Molybdän, Wolfram, Mangan, Rhenium, Zink, Cadmium, Blei, Aluminium, Zinn, Phosphor, Arsen, Antimon, Bismut und Seltenerdmetalle, sowie
iii) von 0 bis 5 Gew.-%, vorzugsweise von 0,1 bis 3 Gew.-%, bezogen auf i) einer Verbindung auf der Basis eines Alkalimetalles oder eines Erdalkalimetalles, enthält.

7. Verfahren nach Anspruch 6 wobei, wenn als Komponente i) eine Verbindung auf der Basis von nur Ruthenium oder Rhodium oder Ruthenium und Rhodium oder Nickel und Rhodium gewählt wird, der Promotor ii) gewünschtenfalls entfallen kann und die Komponente i) nicht auf der Basis von Eisen besteht, wenn die Komponente ii) Aluminium ist.

8. Verfahren nach den Ansprüchen 1 bis 4, wobei man in Schritt a) einen Katalysator verwendet, der
i) eine Verbindung auf der Basis von Eisen,
ii) von 0 bis 5 Gew.-% bezogen auf i) eines Promotors auf der Basis eines Elementes oder 2, 3, 4, 5 oder 6 Elementen ausgewählt aus der Gruppe bestehend aus Aluminium, Silizium, Zirkonium, Mangan, Vanadium und Titan sowie
iii) von 0 bis 5 Gew.-% bezogen auf i) einer Verbindung auf der Basis eines Alkali- oder Erdalkalimetalles, enthält.

9. Verfahren nach den Ansprüchen 1 bis 8, wobei man in Schritt a) zusätzlich ein Verdünnungsmittel verwendet.

10. Verfahren nach Anspruch 9, wobei man als Verdünnungsmittel primäre, sekundäre oder tertiäre Amine, Ammoniak oder Alkohole oder deren Gemische verwendet.

11. Verfahren nach Anspruch 9 oder 10, wobei man das Verdünnungsmittel zwischen den Schritten a) und b) abtrennt.

12. Verfahren nach den Ansprüchen 1 bis 11, wobei man in Schritt a) zusätzlich basische Stoffe zusetzt.

13. Verfahren nach Anspruch 12, wobei man als basische Stoffe Hydroxide, Carbonate oder Alkoholate der Alkali- oder Erdalkalimetalle oder deren Gemische verwendet.

14. Verfahren nach den Ansprüchen 1 bis 13, wobei man nach den Schritten d1) oder c2) das Adipodinitril in Schritt a) zurückführt.

15. Verfahren nach den Ansprüchen 1 bis 13, wobei man nach den Schritten d1) oder c2) das Adipodinitril zu Hexamethylendiamin hydriert.

16. Verfahren nach den Ansprüchen 1 bis 15, wobei der Gehalt in dem in Schritt a) eingesetzten Adipodinitril an 1-Amino-2-cyano-cyclopenten unter 5000 Gew.-ppm bezogen auf Adipodinitril liegt.

17. Verfahren nach den Ansprüchen 1 bis 16, wobei man in Schritt d1) in den Sumpf eine organische oder anorganische Säure zuführt.

18. Verfahren nach den Ansprüchen 1 bis 17, wobei man das nach den Schritten d1) oder c2) erhaltene Adipodinitril mit einer organischen oder anorganischen Säure reinigt.

## Claims

1. A process for the coproduction of 6-aminocapronitrile and hexamethylenediamine starting from adiponitrile, which comprises the steps of
a) hydrogenating adiponitrile in the presence of a catalyst comprising an element of the eighth transition group as catalytically active component, to obtain a mixture comprising 6-aminocapronitrile, hexamethylenediamine, adiponitrile and high boilers,
b) distillatively removing hexamethylenediamine from the mixture comprising 6-aminocapronitrile, hexamethylenediamine, adiponitrile and high boilers, and either
c1) distillatively removing 6-aminocapronitrile, and then
d1) distillatively removing adiponitrile,
or
c2) simultaneously distillatively removing 6-aminocapronitrile and adiponitrile into separate fractions,
**characterized by** base of column temperatures below 185°C in steps d1) or c2).

2. A process as claimed in claim 1, wherein base of column temperatures are below 180°C in steps d1) or c2).

3. A process as claimed in claim 1 or 2, wherein the catalyst in step a) comprises iron, cobalt, nickel, ruthenium or rhodium or mixtures thereof as catalytically active element.

4. A process as claimed in any of claims 1 to 3, wherein the catalyst in step a) comprises iron, cobalt or nickel or mixtures thereof as catalytically active element.

5. A process as claimed in any of claims 1 to 4, wherein the catalyst in step a) is based on Raney nickel or Raney cobalt or mixtures thereof.

6. A process as claimed in any of claims 1 to 4, wherein the catalyst used in step a) comprises
i) a compound based on a metal selected from the group consisting of nickel, cobalt, iron, ruthenium and rhodium,
ii) from 0.01 to 25% by weight, preferably from 0.1 to 5% by weight, based on a), of a promoter based on a metal selected from the group consisting of palladium, platinum, iridium, osmium, copper, silver, gold, chromium, molybdenum, tungsten, manganese, rhenium, zinc, cadmium, lead, aluminum, tin, phosphorus, arsenic, antimony, bismuth and rare earth metals, and also
iii) from 0 to 5% by weight, preferably from 0.1 to 3% by weight, based on i), of a compound based on an alkali metal or an alkaline earth metal.

7. A process as claimed in claim 6, wherein, if a compound based on only ruthenium or rhodium or ruthenium and rhodium or nickel and rhodium is chosen as component i), the promoter ii) can, if desired, be dispensed with, and the component i) shall not be based on iron when the component ii) is aluminum.

8. A process as claimed in any of claims 1 to 4, wherein the catalyst used in step a) comprises
i) a compound based on iron,
ii) from 0 to 5% by weight based on i) of a promoter based on an element or 2, 3, 4, 5 or 6 elements selected from the group consisting of aluminum, silicon, zirconium, manganese, vanadium and titanium, and also
iii) from 0 to 5% by weight based on i) of a compound based on an alkali or alkaline earth metal.

9. A process as claimed in any of claims 1 to 8, wherein a diluent is additionally used in step a).

10. A process as claimed in claim 9, wherein the diluent used is selected from primary, secondary or tertiary amines, ammonia or alcohols or mixtures thereof.

11. A process as claimed in claim 9 or 10, wherein the diluent is removed between steps a) and b).

12. A process as claimed in any of claims 1 to 11, wherein basic substances are additionally added in step a).

13. A process as claimed in claim 12, wherein the basic substances used are hydroxides, carbonates or alkoxides of the alkali or alkaline earth metals or mixtures thereof.

14. A process as claimed in any of claims 1 to 13, wherein adiponitrile is recycled into step a) after steps d1) or c2).

15. A process as claimed in any of claims 1 to 13, wherein adiponitrile is hydrogenated to hexamethylenediamine after steps d1) or c2).

16. A process as claimed in any of claims 1 to 15, wherein the 1-amino-2-cyanocyclopentene content of the adiponitrile used in step a) is below 5000 weight ppm based on adiponitrile.

17. A process as claimed in any of claims 1 to 16, wherein an organic or inorganic acid is added to the bottom product in step d1).

18. A process as claimed in any of claims 1 to 17, wherein the adiponitrile obtained in steps d1) or c2) is purified with an organic or inorganic acid.

## Revendications

1. Procédé de préparation simultanée de 6-aminocapronitrile et d'hexaméthylènediamine, à partir d'adipodinitrile, comprenant les étapes
a) d'hydrogénation d'adipodinitrile en présence d'un catalyseur, qui, comme composant catalytiquement actif, contient un élément du huitième groupe secondaire, avec obtention d'un mélange contenant du 6-aminocapronitrile, de l'hexaméthylènediamine, de l'adipodinitrile et des substances à point d'ébullition élevé,
b) de séparation par distillation d'hexaméthylènediamine à partir du mélange contenant du 6-aminocapronitrile, de l'hexaméthylènediamine, de l'adipodinitrile et des substances à point d'ébullition élevé, et
c1) ou bien de séparation par distillation de 6-aminocapronitrile, et ensuite
d1) de séparation par distillation d'adipodinitrile, ou bien
c2) de séparation par distillation simultanée de 6-aminocapronitrile et d'adipodinitrile en fractions séparées,
**caractérisé en ce que**, dans les étapes d1) ou c2), les températures de fond de cuve sont inférieures à 185°C.

2. Procédé suivant la revendication 1, dans lequel les températures de fond de cuve sont inférieures à 180°C dans les étapes d1) ou c2).

3. Procédé suivant l'une des revendications 1 et 2, dans lequel le catalyseur contient dans- l'étape a), comme élément catalytiquement actif, du fer, du cobalt, du nickel, du ruthénium ou du rhodium ou leurs mélanges.

4. Procédé suivant l'une des revendications 1 à 3, dans lequel le catalyseur contient dans l'étape a), comme élément catalytiquement actif, du fer, du cobalt ou du nickel ou leurs mélanges.

5. Procédé suivant l'une des revendications 1 à 4, dans lequel le catalyseur dans l'étape a) est à base de nickel de Raney ou de cobalt de Raney ou de leurs mélanges.

6. Procédé suivant l'une des revendications 1 à 4, dans lequel on utilise dans l'étape a) un catalyseur qui contient
i) un composé à base d'un métal, choisi parmi le groupe constitué du nickel, du cobalt, du fer, du ruthénium et du rhodium,
ii) de 0,01 à 25 % en poids, de préférence de 0,1 à 5 % en poids, par rapport à i), d'un promoteur à base d'un métal choisi parmi le groupe constitué du palladium, du platine, de l'iridium, de l'osmium, du cuivre, de l'argent, de l'or, du chrome, du molybdène, du tungstène, du manganèse, du rhénium, du zinc, du cadmium, du plomb, de l'aluminium, de l'étain, du phosphore, de l'arsenic,. de l'antimoine, du bismuth et des métaux des terres rares, ainsi que
iii) de 0 à 5 % en poids, de préférence de 0,1 à 3 % en poids, par rapport à i), d'un composé à base d'un métal alcalin ou d'un métal alcalino-terreux.

7. Procédé suivant la revendication 6, dans lequel, lorsqu'on choisit, comme composant i), un composé à base de seulement du ruthénium ou du rhodium ou du ruthénium et du rhodium ou du nickel et du rhodium, le promoteur ii) peut éventuellement disparaître et le composant i) n'est pas constitué à base de fer, lorsque le composant ii) est de l'aluminium.

8. Procédé suivant l'une des revendications 1 à 4, dans lequel on utilise dans l'étape a) un catalyseur qui contient
i) un composé à base de fer,
ii) de 0 à 5 % en poids, par rapport à i), d'un promoteur à base d'un élément ou de 2, 3, 4, 5 ou 6 éléments choisis parmi le groupe constitué de l'aluminium, du silicium, du zirconium, du manganèse, du vanadium et du titane, ainsi que
iii) de 0 à 5 % en poids, par rapport à i), d'un composé à base d'un métal alcalin ou d'un métal alcalino-terreux.

9. Procédé suivant l'une des revendications 1 à 8, dans lequel on utilise, dans l'étape a), un agent diluant en supplément.

10. Procédé suivant la revendication 9, dans lequel on utilise, comme diluant, des amines primaires, secondaires ou tertiaires, de l'ammoniac ou des alcools ou leurs mélanges.

11. Procédé suivant l'une des revendications 9 et 10, dans lequel on isole le diluant entre les étapes a) et b).

12. Procédé suivant l'une des revendications 1 à 11, dans lequel, dans l'étape a), on ajoute en supplément des substances basiques.

13. Procédé suivant la revendication 12, dans lequel on utilise, comme substances basiques, des hydroxydes, des carbonates ou des alcoolates des métaux alcalins ou alcalino-terreux ou leurs mélanges.

14. Procédé suivant l'une des revendications 1 à 13, dans lequel, après les étapes d1) ou c2), on retourne l'adipodinitrile dans l'étape a).

15. Procédé suivant l'une des revendications 1 à 13, dans lequel, après les étapes d1) ou c2), on hydrogène l'adipodinitrile pour former de l'hexaméthylènediamine.

16. Procédé suivant l'une des revendications 1 à 15, dans lequel la teneur en 1-amino-2-cyano-cyclopentène dans l'adipodinitrile mis en oeuvre dans l'étape a) est inférieure à 5.000 ppm en poids, par rapport à l'adipodinitrile.

17. Procédé suivant l'une des revendications 1 à 16, dans lequel, dans l'étape d1), on amène dans le fond de cuve un acide organique ou inorganique.

18. Procédé suivant l'une des revendications 1 à 17, dans lequel on purifie l'adipodinitrile obtenu selon les étapes d1) ou c2) avec un acide organique ou inorganique.
